# EUROPÄISCHE PATENTANMELDUNG

(11) **EP 0 724 883 A1**
(43) Veröffentlichungstag der Anmeldung: **07.08.1996**
(21) Anmeldenummer: 95890023.5
(22) Anmeldetag: 31.01.1995
(51) Int. Cl.: A61K 35/78, A61K 7/04, A61K 7/043

(54) **Mittel zur Behandlung und Präventivpflege von Hufen**

(71) Anmelder: Haerpfer-Horn, Herta, Mag., A-1040 Wien (AT)
(72) Erfinder: Haerpfer-Horn, Herta, Mag., A-1040 Wien (AT)
(74) Vertreter: Itze, Peter, Dipl.-Ing.

(57) **Zusammenfassung**

Die Erfindung bezieht sich auf ein Mittel zur Behandlung und Pflege von Hufen, insbesondere der Weichhornteile auf der Lauffläche von Pferdehufen, wobei bakterizid wirkende Pflanzenessenzen in pflanzlichen Ölen gelöst sind.

## Beschreibung

Die Erfindung bezieht sich auf ein Mittel zur Behandlung und Präventivpflege von Hufen, insbesondere der Weichhornteile auf der Lauffläche von Pferdehufen.

Beim Pferd ist ein einwandfreier Bewegungsablauf untrennbar mit dem funktionierenden Hufmechanismus verbunden. Der Hufmechanismus funktioniert aber nur bei einer ausreichenden Elastizität des Hornschuhs, der aus der Hornsohle, der Hornwand und dem Hornstrahl besteht. Für diese Aufgabe muß der doppelschenkelige, aus einer weichen Hornmasse bestehende Strahl kräftig und markant ausgeprägt sein. Die Elastizität des Hornstrahls ergibt sich aufgrund der Beschaffenheit; er dehnt sich und zieht sich wieder zusammen. Dies fördert die Durchblutung, regt das Hornwachstum an und dämpft die Härte des Aufsetzens. Die zweite wichtige Funktion des Hufstrahls liegt im Schutz der empfindlichen Huflederhaut.

Bei Auftreten von Strahlfäule (Zerfall des Horns oder Hufstrahls) 'fault' der Hornstrahl weg - es kommt zu einem schmierigen Zerfall des Hornstrahls, wobei dieser in der ersten Phase zerrissen und zerklüftet wird und in der Folgephase schrumpft, d.h. daß er effektiv an Volumen verliert. Dies hat jedoch sofort eine Störung des Hufmechanismus zur Folge, was sich wiederum auf den gesamten Bewegungsablauf negativ auswirkt und in den meisten Fällen zur Lahmheit des betroffenen Pferdes führt. Daneben treten Durchblutungsstörungen auf, es kommt zum Verlust der Schutzfunktion für die Huflederhaut, die sich sehr leicht entzünden kann. Entzündungen der Huflederhaut sind äußerst schmerzhaft und machen es dem Pferd unmöglich, den betroffenen Huf voll zu belasten. Darüber hinaus bilden Entzündungen der Huflederhaut den Ausgangspunkt für unheilbare Erkrankungen wie z.B. die Hufrehe.

Als häufigste Ursache kommen in den meisten Fällen Stallungen in Betracht, in denen nicht regelmäßig ausgemistet wird. Durch langes Stehen der Pferde auf feuchter Streu durchweicht deren Nässe den Hornstrahl mit der Folgewirkung, daß Fäulnisbakterien in das geschädigte Horn eindringen und es zersetzen. Gefördert wird der Gewebszerfall durch die Fäulnisbakterien durch die Bildung von Taschen, die sich in der ersten Phase unterhalb des Hornstrahls bilden und in denen sich die übelriechende Gewebszersetzung am besten hält. Des weiteren sind für diese Huferkrankung besonders solche Pferde anfällig, deren Hufstrahl von vornherein infolge von Durchblutungsstörungen der Huflederhaut von schlechter Beschaffenheit ist.

Strahlfäule ist heilbar, doch steht zur direkten Behandlung nur die hochgiftige anorganische Metallverbindung Kupfervitriol CusO₄ zur Bekämpfung der die Fäulnis hervorrufenden Bakterien zur Verfügung, durch deren Verwendung es zwar zu einer momentanen Besserung kommt, ein Wiederbefall jedoch nicht verhindert werden kann.

Der Erfindung liegt die Aufgabe zugrunde, ein - aus ausschließlich naturreinen Substanzen von hoher Qualität bestehendes - Mittel für die örtliche Behandlung akuter Strahlfäule bei Pferden zu schaffen, das den Fäulnisvorgang, d.h. die Gewebszersetzung, stoppt, die Wiederherstellung eines funktionsfähigen Hufstrahls beschleunigt, und dadurch in relativ kurzer Zeit einen einwandfreien Ablauf des Hufmechanismus bewirkt. Darüber hinaus soll das erfindungsgemäße Mittel einen Wiederbefall des Pferdes durch diese Krankheit verhindern.

Diese Aufgabe wird erfindungsgemäß dadurch gelöst, daß bakterizid wirkende Pflanzenessenzen in pflanzlichen Ölen gelöst sind. Durch diese Kombination ergibt sich die Möglichkeit, die außergewöhnlich starken antibakteriellen Wirkstoffe der Essenzen dort einsetzen zu können, wo diese die otpimalsten Ergebnisse bringen. Da das Eindringen der Fäulnisbakterien den Zerfall des Hornstrahls auslöst, steht an erster Stelle die Notwendigkeit der Vernichtung der Fäulnisbakterien, um der Gewebszersetzung Einhalt zu gebieten und somit die Regeneration des Hufstrahls zu bewirken.

Vorteilhafterweise können als bakterzid wirkende Pflanzenessenzen die ätherischen Öle von Bohnenkraut und/oder Cajeput und/oder Eukalyptus citriodora und/oder Lavendel und/oder Knoblauch und/oder Niouli und/oder Tea-Tree eingesetzt sein. Diese Essenzen zählen zu den stärksten bakteriziden Essenzen. Dadurch wird die Auflösung, Verdauung und Neutralisierung der Fäulnisbakterien erzielt, wobei keine gegenseitige Beeinflussung der Wirksamkeit auftrtitt, sondern vielmehr ein synergistischer Effekt auftritt.

Den pflanzlichen Ölen, in denen die hochkonzentrierten ätherischen Öle gelöst sind, wird hier eine besondere Bedeutung zugemessen: durch ihre Wahl als Lösungsmittel wurde ein Weg gefunden, die bakterientötenden Wirkstoffe der ätherischen Öle voll wirksam werden zu lassen. Während die gebräuchlichen Mittel zur Bekämpfung der Strahlfäulnis, wie z.B. das hochgiftige Kupfervitriol, die Bakterien nur an den Stellen vernichten können, an denen ein direkter Kontakt stattfindet - also nur an der Oberfläche des angegriffenen Hornstrahls, werden die Trägeröle von dem Gewebe absorbiert, wodurch die Wirkstoffe der in ihnen gelösten ätherischen Öle bis zum effektiven Bakterienherd, nämlich den unter dem Hornstrahl gebildeten Taschen vordringen und dort mit Erfolg wirksam werden können.

Aus diesem Grund werden in der erfindungsgemäßen Mixtur nur hochwertige, kaltgepreßte und 100% reine Pflanzenöle - Weizenkeimöl und/oder Mandelöl und/oder Jojobaöl - verwendet, die darüber hinaus noch andere, den Krankheitsverlauf positiv beeinflussende Wirkstoffe, wie z.B. entzündungshemmende Myristinsäure des Jojobaöls, beinhalten. Sie regen die Durchblutung an und fördern die Gewebeneubildung. Außerdem bewirken die pflanzlichen Öle eine Rückfettung der Haut und bilden einen Schutzfilm der befallenen Stellen.

In Anbetracht der Folgeerscheinungen des Bakterienbefalls ist es von Vorteil, der erfindungsgemäßen Mixtur zusätzlich solche ätherischen Öle beizugeben, deren Wirkungsspektrum weit über die Beseitigung des Kernübels - den Bakterienbefall - hinausgeht; d.s. solche, die entzündungshemmend und schmerzlindernd wirken - Elemie und/oder Lavendel und/oder Patschouli und/oder Tea Tree -, ferner solche, die der zweiten Ursache für das Auftreten der Strahlfäulnis, der schlechten Durchblutung bzw. den Durchblutungsstörungen der Strahllederhaut, entgegenwirken, d.s. Niouli und/oder Rosmarin und/oder Thymian, die die Durchblutung direkt an den behandelten Stellen stark anregen. Ferner wurden der erfindungsgemäßen Mixtur Essenzen beigegeben, die ein verstärktes Gewebewachstum bewirken und somit den Wiederaufbau des Hornstrahls, damit der Hufmechanismus beim Pferd in relativ schneller Zeit wieder problemlos funktioniert.

Schließlich kann das erfindungsgemäße Mittel zusätzlich noch pflanzliche Essenzen aus Rosmarin und/oder Thymian und/oder Zedernholz und/oder Zimt enthalten. Dadurch wird eine durchblutungsfördernde und geweberegenerierende Wirkung erzielt, wobei überdies die eingetretene Fäulnis gestoppt wird und auch das Neuauftreten von Fäulnis verhindert wird.

Um ein bestmögliches Resultat zu erzielen - einfache Anwendung - schnelle Wirkung - Schutz vor Wiederbefall - wurde bei der erfindungsgemäßen Substanz besonders auf die Qualität der ihr zugrundeliegenden Stoffe geachtet. So werden nur absolut reine ätherische Öle verwendet, die in bezug auf ihre Reinheit und Qualität weit über die Anforderungen des Österreichischen Arzneibuches (Ö.A.B.) hinausgehen.

Die Wirkungen der einzelnen Substanzen sind in Tabelle I zusammengestellt.

Im folgenden sind zwei Zusammensetzungsbeispiele wiedergegeben, von denen sich das Beispiel 1 auf ein Mittel zur Vorbeugung und Beispiel 2 auf ein Mittel zur Wiederherstellung eines gesunden Hornstrahles bezieht.

### Beispiel 1:

| Äth.Öle/Pflanzenöle | in % | i.v. 70: 30/100 ml |
|---|---|---|
| Bohnenkraut | 15 | 4,5 |
| Eukalyptus citriodora | 15 | 4,5 |
| Lavendel | 10 | 3,0 |
| Knoblauch | 8 | 2,4 |
| Rosmarin | 10 | 3,0 |
| Tea Tree | 10 | 3,0 |
| Thymian | 12 | 3,6 |
| Zedernholz | 10 | 3,0 |
| Zimt | 10 | 3,0 |
| | | 30,0 ml |
| Weizenkeimöl | 64,3 % | 45,0 ml |
| Jojobaöl | 35,7 % | 25,0 ml |
| | | 100,0 ml |

### Beispiel 2:

| äth.Öle/Pflanzenöle | in % | i.v. 60:40/100 ml |
|---|---|---|
| Bohnenkraut | 11 | 4,4 |
| Cajeput | 4 | 1,6 |
| Elemi | 15 | 6,0 |
| Eukalyptus ctriodora | 8 | 3,2 |
| Lavendel | 16 | 6,4 |
| Knoblauch | 12 | 4,8 |
| Niouli | 6 | 2,4 |
| Patsschouli | 9 | 3,6 |
| Rosmarin | 4 | 1,6 |
| Tea Tree | 5 | 2,0 |
| Thymian | 10 | 4,0 |
| Weizenkeimöl | 50 % | 30,0 ml |
| Mandelöl | 50 % | 30,0 ml |
| | | 100,0 ml |

**Tabelle 1**

| | bakterizid | fäulnisstoppend | entzündungshemmend | duchblutungsfördernd | geweberegenerierend | desinfiziere |
|---|---|---|---|---|---|---|
| Bohnenkraut | XXX | | | | | |
| Cajeput | XXX | | | | | |
| Elemi | | XX | XX | | X | |
| Eukalyptus citriodora | XXX | | | X | XX | |
| Lavendel | XXX | | XXX | X | XXX | |
| Knoblauch | XXX | XXX | | | XXX | |
| Niouli | XXX | | | XX | XX | |
| Patschouli | XX | | XX | | XXX | |
| Rosmarin | | | | XXX | XXX | XXX |
| Tea-Tree | XXX | | XXX | | | |
| Thymian | | | | XXX | XXX | |
| Zedernholz | | | XX | | | |
| Zimt | | XX | | | | |
| WZK | | | | X | XX | |
| Jojobaöl | | | | | | |

## Patentansprüche

1. Mittel zur Behandlung und Pflege von Hufen, insbesondere der Weichhornteile auf der Lauffläche von Pferdehufen, dadurch gekennzeichnet, daß bakterizid wirkende Pflanzenessenzen in pflanzlichen Ölen gelöst sind.

2. Mittel nach Anspruch 1, dadurch gekennzeichnet, daß als bakterizid wirkende Pflanzenessenzen die ätherischen Öle von Bohnenkraut und/oder Cajeput und/oder Eucalyptus citriodora und/oder Lavendel und/oder Knoblauch und/oder Niouli und/oder Tea Tree eingesetzt sind.

3. Mittel nach Anspruch 1 oder 2, dadurch gekennzeichnet, daß als pflanzliche Öle, kaltgepreßte und völlig reine Öle eingesetzt sind.

4. Mittel nach Anspruch 3, dadurch gekennzeichnet, daß als pflanzliche Öle Weizenkeimöl und/oder Mandelöl und/oder Jojobaöl verwendet ist.

5. Mittel nach einem der Ansprüche 1 bis 4, dadurch gekennzeichnet, daß es zusätzlich noch entzündungshemmend und/oder schmerzlindernd wirkende Pflanzenessenzen enthält.

6. Mittel nach Anspruch 5, daudurch gekennzeichnet, daß als entzündungshemmende und/oder schmerzlindernde Mittel zusätzlich zu Lavendel und/oder Tea Tree noch Elemie und/oder Patschouli zugesetzt ist.

7. Mittel nach einem der Ansprüche 1 bis 6, dadurch gekennzeichnet, daß es zusätzlich noch pflanzliche Essenzen aus Rosmarin und/oder Thymian und/oder Zedernholz und/oder Zimt enthält.
